# EUROPÄISCHE PATENTANMELDUNG

(11) **EP 1 095 694 A2**
(43) Veröffentlichungstag der Anmeldung: **02.05.2001**
(21) Anmeldenummer: 00250356.3
(22) Anmeldetag: 26.10.2000
(51) Int. Cl.: B01D 71/02, B01D 69/02, B01D 53/22, G01N 27/40

(54) **Gasdurchlässige Membran und deren Verwendung zur Steuerung des Gasdurchganges**

(30) Priorität: 26.10.1999 DE 19952725
(71) Anmelder: Institut für Angewandte Chemie Berlin-Adlershof E.V., 12489 Berlin (DE)
(72) Erfinder: Noack, Manfred, Dr., 12683 Berlin (DE); Kölsch, Peter, Dr., 10367 Berlin (DE); Caro, Jürgen, Dr., 13129 Berlin (DE); Weh, Kornelia, 12489 Berlin (DE)
(74) Vertreter: Walter, Wolf-Jürgen

(57) **Zusammenfassung**

Die Erfindung betrifft eine Membran aus Molekularsieb-Kristallen, bei der ein unterschiedlicher Gasdurchgang durch optisches Schalten erreicht werden kann. Erfindungsgemäß besteht die Membran aus einem porösen anorganischen Trägermaterial und einer Funktionsschicht, wobei die anorganische Funktionsschicht an der Oberfläche des porösen Trägers als eine dünne, malt Licht der Wellenlänge 300 bis 500 nm durchstrahlbare Schicht eines Molekularsiebes mit definiertem dreidimensionalen Porenaufbau angeordnet ist und wobei die Molekularsiebschicht bei mindestens einer Porenart eine Weite von 0,5 bis 1 nm hat und in den Poren aromatische, photochemisch isomerisierbare Moleküle mit -N=N- oder -C=C-Gruppen enthält.

## Beschreibung

Gasdurchlässige Membran und deren Verwendung zur Steuerung des Gasdurchganges

Die Erfindung betrifft eine Membran aus Molekularsieb-Kristallen, bei der ein unterschiedlicher Gasdurchgang durch optisches Schalten erreicht werden kann.

Bekannt ist eine photoinduzierte Potentialänderung, verbunden mit der Veränderung der Ionenpermeation durch eine Polymermembran, die Azobenzen-modifizierte Kronenether enthält [J.Anzai, T. Osa, Tetrahedron 50 (1994) 4039-4070]. Dabei wird ein Lösungs-Diffusions-Mechanismus wirksam, der zu einem Quellen der Membran und damit veränderten Eigenschaften führt.

Der Erfindung liegt die Aufgabe zugrunde, neue Membranen bereitzustellen, die einen unterschiedlichen Gasdurchgang bei definierten Porengrößen ermöglichen.

Erfindungsgemäß besteht die Membran aus einem porösen anorganischen Trägermaterial und einer Funktionsschicht, wobei die anorganische Funktionsschicht an der Oberfläche des porösen Trägers als eine dünne, mit Licht der Wellenlänge 300 bis 500 nm durchstrahlbare Schicht eines Molekularsiebes mit definiertem dreidimensionalen Porenaufbau angeordnet ist, und wobei die Molekularsiebschicht bei mindestens einer Porenart eine Weite von 0,5 bis 1 nm hat und in den Poren aromatische, photochemisch isomerisierbare Moleküle mit -N=N- oder -C=C-Gruppen enthält.

Mit Hilfe dieses Aufbaus kann bei Bestrahlung der Funktionsschicht eine photochemisch induzierte Isomerisierung der in den definierten Poren der Schicht (Wirt) adsorbierten aromatischen Moleküle (Gast) erfolgen, wodurch das Transportverhalten für durchdringende Gasmoleküle verändert wird. Die Photoisomerisierung versetzt die -N=N- bzw. -C=C-Verbindungen in den trans- oder cis-Zustand. Diese Molekülzustände haben einen unterschiedlichen Raumbedarf, eine unterschiedliche Polarität und eine unterschiedliche Geometrie und können deshalb den Durchtritt eines Gases durch die Membran beeinflussen. Dabei kann sowohl die Menge des Gasflusses durch die Membran als auch die Zusammensetzung des hindurchtretenden Gases im Falle von Gemischen gesteuert werden.

Das anorganische Trägermaterial kann aus der Gruppe poröse Gläser, Keramiken, Sintermetalle oder Metallgazen ausgewählt werden, wobei wegen der Ähnlichkeit der Ausdehungskoeffizienten von Gläsern oder Keramiken mit Molekularsieben Gläser und Keramiken bevorzugt sind.

Das Molekularsieb kann aus der Gruppe Faujasit, EMT, MFI einschließlich ZSM-5 und Silikalith, AFY, AFR, GME, MOR und OFF ausgewählt sein. Bevorzugt sind Faujasit (mit den isostrukturellen Faujasiten Linde X und Linde Y) sowie solche vom Typ EMT und MFI einschließlich ZSM-5 und Silikalith.

Die aromatischen, photochemisch isomerisierbaren Moleküle sind vorzugsweise solche der Formel (I) oder (II) 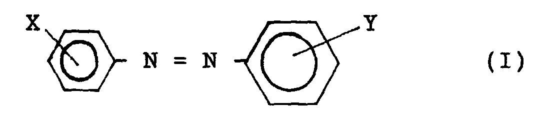 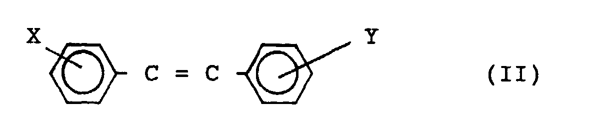 worin X und Y, die gleich oder verschieden sein können, jeweils Wasserstoff, C₁-C₅-Alkyl, Amino, Amido, C₁-C₅-Alkylamino, Nitro, Halogen, Hydroxy, C₁-C₅-Alkoxy, C₁-C₅-Alkylcarbonyl oder mit Halogen substituiertes C₁-C₅-Alkyl oder C₁-C₅-Alkoxy darstellen.

Bevorzugt sind im Sinne der Erfindung einkernige Derivate mit Substituenten, die weder die Größe des Moleküls über den vorgegebenen Rahmen der Porengröße in der Funktionssschicht erweitern, noch die photochemische Schaltbarkeit des cis-trans-Überganges wesentlich verändern, d.h. die Reversibilität und die Lage des photochemischen Gleichgewichtes bei Raum- und Strahltemperatur. Die Substituenten können Einfluß auf die adsorptive Wechselwirkung der Moleküle der Formeln (I) oder (II) mit der Porenoberfläche des Molekularsiebes nehmen oder selbst eine chemische Bindung zu dieser Oberfläche eingehen.

Bevorzugt sind Wasserstoff, Methyl, Ethyl, Hydroxy, Methoxy sowie Halogenreste wie Fluor oder Chlor.

Besonders bevorzugt sind X und Y in den Formeln (I) oder (II) Wasserstoff, wobei eine insbesondere bevorzugte Verbindung Azobenzen ist.

Die genannten Gastmoleküle können adsorptiv oder reaktiv an der Porenoberfläche der anorganischen Molekularsiebschicht gebunden werden. Das Einbringen kann über die Flüssig- oder Gasphase erfolgen. Dabei erfolgt die reaktive Bindung (chemische Immobilisierung zwischen OH-Oberflächengruppen oder Kationen des Molekularsiebes mit den reaktiven Substituenten des Gastmoleküls und zwar einseitig, um die Schaltbarkeit des Moleküls nicht einzuschränken. Für die reaktive Bindung sollten X und Y in den obigen Formeln (I) und (II) nicht gleichzeitig Wasserstoff, Alkyl oder Halogen sein.

Die Beladung mit dem Gastmolekül ist so einzustellen, daß keine unbeeinflußten Porensysteme bestehen und zugleich keine Blockade aller Kreuzungspunkte erfolgt. Sie liegt daher im allgemeinen je nach Typ des Zeoliths bei 1 bis 20 Gew-%.

Die Herstellung der Molekularsiebmembran erfolgt nach bekannten Verfahren, beispielsweise durch hydrothermale einstufige oder zweistufige Synthese oder durch Kristallisation in der Gasphase.

Bei der hydrothermalen Synthese verläuft der Kristallisationsprozeß des Syntheseansatzes im wäßrigen System unter Einfluß erhöhter Temperatur und Zeitdauer. Es bilden sich mehrlagige Kristallschichten aus. Bei der zweistufigen Synthese wird die Keimbildung separat durchgeführt. Die Keimkristalle werden mit oder ohne Hilfsstoffe am Träger abgeschieden und thermisch nachbehandelt. In einer neuen Syntheselösung wächst anschließend unter hydrothermalen Bedingungen aus den Keimen auf dem Träger eine dichte, dünne Kristallschicht.

Bei der Kristallisation in der Gasphase wird das Ausgangsmaterial für die spätere Kristallschicht als Gel auf einem Träger abgeschieden und anschließend im geschlossenen System im Gleichgewicht mit Wasser- und Templat-Dämpfen bei erhöhter Temperatur zur Kristallisation gebracht.

Der poröse Träger und die Funktionsschicht sind durchlässig vorzugsweise für polare, polarisierbare oder unpolare Moleküle als Gase und Dämpfe bis zu einem Molekulargewicht von 200.

Die Steuerung des Gasdurchflusses erfolgt ohne mechanische Schaltelemente. Die erfindungsgemäße Membran läßt sich bis auf die Größe von Einkristallen in den µm-Bereich miniaturisieren.

Da das Licht definierter Wellenlänge über Lichtleiter zugeführt werden kann, können viele Membranen von einer Bestrahlungsapparatur aus geschaltet werden. Die Lichtzuführung erfolgt beispielsweise durch Koppelung der Bestrahlungsapparatur, bestehend aus Lichtquelle und Filtern, mit dem Membranmodul über einen Lichtleiter.

Eine schnelle Änderung der Transporteigenschaften der Membran kann durch Wechsel der Bestrahlungswellenlängen bei Dauerbestrahlung erreicht werden.

Die erfindungsgemäße Membran eignet sich als Mikroventil für die Mikroreaktionskinetik, in Kombination mit einem Sensor verursacht sie eine veränderbare Sensorcharakteristik, zum Einbau in Gaschromatographen als Null-Längen-Säule mit veränderbarer Trenncharakteristik sowie zur Simulation eines Tag/Nacht-Zyklus in Anlehnung an Assimilation/Dissimilation z.B. in belebten Räumen oder zur Steuerung von Reifeprozessen.

So kann z.B. bei letztgenannter Verwendungsmöglichkeit zur Änderung der Transporteigenschaften der Membran nur die Wellenlänge für das instabilere Photoisomere eingestrahlt werden (auch gefiltertes Tageslicht). Der Zustand mit dem stabileren Photoisomeren stellt sich nach Abschalten der Bestrahlung (oder in der Nacht durch thermische Relaxation) langsam selbst ein.

Die Verwendung ist ebenfalls Gegenstand der Erfindung.

Die Erfindung soll nachstehend durch Beispiele näher erläutert werden.

### Beispiel 1

Auf einen offenporigen keramischen Träger mit einer Porengröße von 5-1000 nm wird eine Zeolithschicht des Typs FAU nach der zweistufigen hydrothermalen Synthese aufkristallisiert. Der molare Ansatz der Kristallkeimbildung ist z.B.
1 Al₂O₃ : 3,6 SiO₂ : 0,04 Na₂O : 1,6 TMAOH : 250 H₂O (TMAOH = Tetramethylammoniumhydroxid)
Nach 100-300 h bei 100 °C werden Faujasitkeime von 50-200 nm Größe erhalten. Der keramische Träger wird mit einem kationischen Polymer umgeladen, und anschließend werden die Keimkristalle an ihm abgeschieden.

Der molare Ansatz für die Kristallschichtbildung ist z.B.
1 Al₂O₃ : 10 SiO₂ : 14 Na₂O : 850 H₂O
Die Bildung der 1-3 µm Faujasit-Kristallschicht erfolgt bei 90 °C in 10 bis 40 Stunden.

Die Zeolithschicht hat eine Porengröße von 0,74 nm. Durch einen Gasphasenprozess werden die Hohlräume des Zeolithen mit Azobenzen gefüllt. Die Faujasit-Membranschicht wird bei 150 °C über 16 h bei 0,001 mbar entwässert. Anschließend erfolgt die Azobenzenbeladung bei 70 °C bei autogenem Druck über 1-5 Stunden über die Gasphase. Der Beladungsgrad beträgt 12 Gew-%.

Die so präparierte erfindungsgemäße Membran wird in eine Permeationsapparatur mit Belichtung eingebaut und einem Differenzgasdruck von 1 bar iso-Butan als Einzelgas ausgesetzt. Bei Belichtung der Membran mit 500 mW/cm² mit Licht der Wellenlänge 440 ma beträgt die Flußdichte des i-Butans durch die Membran hindurch 20 l/m²h bar. Wird die Wellenlänge der Belichtung auf 360 nm verändert, sinkt innerhalb von 30 Sekunden der Fluß des i-Butans durch die Membran auf 4 l/m²h bar.

### Beispiel 2

Es wird eine Membran wie im Beispiel 1 präpariert und als Sensor zur Bestimmung einer Gaszusammensetzung benutzt. Bei einem Gasgemisch I aus 95 Vol-% Wasserstoff und 5 Vol-% i-Butan passiert bei einer Belichtung der Membran mit einer Wellenlänge von 440 nm eine Gasmenge von 45 l/m²h bar (100 %) die Membran. Bei Änderung der Belichtungswellenlänge auf 360 nm sinkt der Fluß nur auf 43,2 l/m²h bar (96 %), da der Wasserstofffluß durch die optische Schaltung kaum beeinflußt wird. Besteht das Gasgemisch II aus 95 Vol-% i-Butan und 5 Vol-% Wasserstoff, beträgt der Fluß durch die Membran 20,5 l/m²h bar bei 440 nm und nur 4,5 l/m²h bar (22 %) bei der Belichtungswellenlänge 360 nm, da der i-Butan-Fluß dieses Gasgemisches II durch die optische Schaltung stark vermindert wird. Aus der relativen Abnahme des Durchflusses läßt sich rechnerisch die Gaszusammensetzung bestimmen.

### Beispiel 3

Auf einen keramischen Träger des Beispiels 1 wird eine Zeolithschicht des MFI-Typs in zweistufiger hydrothermaler Synthese aufkristallisiert. Molarer Ansatz für die Keimkristallbildung
9 TPAOH : 0,1 Na₂O : 25 SiO₂ : 480 H₂O : 100 EthOH (TPAOH = Tetrapropylammoniumhydroxid)
Bei 100 °C entstehen im Zeitraum von 20-50 h 60-100 nm große MFI-Kristallkeime. Die MFI-Keime wurden am umgeladenen Träger angelagert und bei 550 °C nachbehandelt. In einem Syntheseansatz der molaren Zusammensetzung
28 Na₂O : 1 Al₂O₃ : 100 SiO₂ : 4000 H₂O
erfolgt bei 180 °C über einen Zeitraum von 10-40 h die Bildung der dichten 2-5 µm dicken MFI-Kristallschicht.

Dabei erhält man eine Funktionsschicht mit Porengrößen von 0,55 nm. Diese Funktionsschicht wird über die Gasphase in gleicher Weise mit 1 bis 3 Gew-% Azobenzen beladen. Permeiert über diese Membran ein 1:1 Wasserstoff/n-Butan-Gemisch, bezogen auf das Volumen der Gase, so läßt sich die Wasserstoffselektivität bei 360 nm-Bestrahlung (cis-Zustand des Azobenzens) gegenüber dem trans-Zustand bei 440 nm verdoppeln.

### Beispiel 4

Auf einem keramischen Träger wird eine Faujasit-Schicht nach Beispiel 1 kristallisiert. Die Beladung der Funktionsschicht mit 10 Gew-% trans-Stilben erfolgt über die Gasphase bei 80 °C über 4 h.

Wird das trans-Stilben mit einer Wellenlänge von 310 nm zum cis-Isomer geschaltet und ein Wasserstoff-Methan-Gemisch überströmt die Membran, so sinkt der Methangehalt im Permeat um den Faktor 4. Beim Abschalten der optischen Anregung relaxiert das cis-Stilben schnell zum stabileren trans-Stilben, was eine Erhöhung des Methangehaltes im Permeat auf den Ausgangswert verursacht.

## Patentansprüche

1. Gasdurchlässige Membran, bestehend aus einem porösen anorganischen Trägermaterial und einer Funktionsschicht, dadurch gekennzeichnet, daß die anorganische Funktionsschicht an der Oberfläche des porösen Trägers als eine dünne, mit Licht der Wellenlänge 300 bis 500 nm durchstrahlbare Schicht eines Molekularsiebes mit definiertem dreidimensionalen Porenaufbau angeordnet ist, wobei die Molekularsiebschicht bei mindestens einer Porenart eine Weite von 0,5 bis 1 nm hat und in den Poren aromatische, photochemisch isomerisierbare Moleküle mit -N=N- oder -C=C-Gruppen enthält.

2. Membran nach Anspruch 1, dadurch gekennzeichnet, daß das anorganische Trägermaterial aus der Gruppe poröse Gläser, Keramiken, Sintermetalle oder Metallgazen ausgewählt ist.

3. Membran nach Anspruch 1, dadurch gekennzeichnet, daß das Molekularsieb aus der Gruppe ausgewählt ist, bestehend aus Faujasit, EMT, MFI einschließlich ZSM-5 und Silikalith, AFY, AFR, GME, MOR , OFF und Gemische davon.

4. Membran nach Anspruch 1, dadurch gekennzeichnet, daß die aromatischen, photochemisch isomerisierbaren Moleküle solche der Formel (I) oder (II) 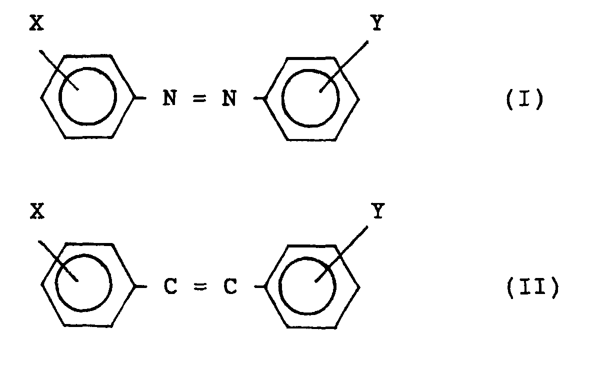 sind, worin X und Y, die gleich oder verschieden sein können, jeweils Wasserstoff, C₁-C₅-Alkyl, Amino, Amido, ₁-C₅-Alkylamino, Nitro, Halogen, Hydroxy, C₁-C₅-Alkoxy, C₁-C₅-Alkylcarbonyl oder mit Halogen substituiertes C₁-C₅-Alkyl oder C₁-C₅-Alkoxy darstellen.

5. Membran nach Anspruch 1, dadurch gekennzeichnet, daß das aromatische, photochemisch isomerisierbare Molekül Azobenzen ist.

6. Membran nach Anspruch 1, dadurch gekennzeichnet, daß das aromatische, photochemisch isomerisierbare Molekül Stilben ist.

7. Membran nach Anspruch 1, dadurch gekennzeichnet, daß der poröse Träger und die Funktionsschicht durchlässig sind für polare, polarisierbare oder unpolare Moleküle als Gase und Dämpfe bis zu einem Molekulargewicht von 200.

8. Verwendung einer Membran aus einem porösen anorganischen Trägermaterial und einer anorganischen Funktionsschicht, wobei die Funktionsschicht an der Oberfläche des Trägers als eine dünne Schicht eines Molekularsiebes mit definiertem dreidimensionalen Porenaufbau angeordnet ist und die Schicht bei mindestens einer Porenart eine Weite von 0,5 bis 1 nm hat und in den Poren aromatische, photochemisch isomerisierbare Moleküle mit -N=N- oder -C=C-Gruppen enthält zur Trennung permeierender Gasmoleküle mit einem MG <200 vor oder nach Einstrahlung von unterschiedlichem Licht aus dem Bereich der Wellenlänge 300 bis 500 nm auf die Funktionsschicht.

9. Verwendung nach Anspruch 8, dadurch gekennzeichnet, daß das isomerisierbare Molekül Azobenzen ist und bei einer Wellenlänge von 440 nm in den trans-Zustand und bei 360 nm in den cis-Zustand geschaltet wird.

10. Verwendung nach Anspruch 8 oder 9, dadurch gekennzeichnet, daß die Membran als Mikroventil oder in Kombination mit einem Sensor veränderlicher Charakteristik oder zur Simulation des Tag-Macht-Zyklus zur Steuerung der Raumluftzusammensetzung belebter Räume eingesetzt wird.
